# EUROPEAN PATENT APPLICATION

(11) **EP 1 726 249 A1**
(43) Date of publication of application: **29.11.2006**
(21) Application number: 05720791.2
(22) Date of filing: 15.03.2005
(51) Int. Cl.: A61B 1/00

(54) **ENDOSCOPE BALLOON CONTROL DEVICE**

(30) Priority: 19.03.2004 JP 2004081655; 12.11.2004 JP 2004329522
(71) Applicant: Olympus Corporation, Tokyo 151-0072 (JP)
(72) Inventor: YOSHIDA, T., c/o Olympus IP Services Co. Ltd, Tokyo 192-8512 (JP)
(74) Representative: von Hellfeld, Axel
(86) International application number: PCT/JP2005/004534
(87) International publication number: WO 2005/089626

(57) **Abstract**

Comprising first and second pumps for supplying or discharging air to or from a balloon for fixing attached to peripheral of a tip part of an overtube for an endoscope being inserted therethrough, after a balloon for fixing attached to a peripheral part of a tip part of an inserting unit of the endoscope and the balloon 11 for fixing attached to the peripheral part of the tip part is attached, a timer counter for detecting a gas sending time or a gas drawing time to or from each of the balloons, and a control unit for controlling a pressure inside each of the balloons by operating the first and second pumps based on a detected result by the timer counter.

## Description

### Technical Field

The present invention relates to an endoscope balloon control device, and relates to an endoscope balloon control device which can control pressures inside a balloon provided for a tip peripheral part of an inserting unit of an endoscope and a balloon provided for a tip peripheral part of an inserting unit of an overtube.

### Background Art

Generally, endosocpy has been widely used in an alimentary canal examination. When an inserting unit of an endoscope is inserted in a deep alimentary canal, such as the small intestine in such an endoscope examination, it is difficult to insert the inserting unit into the deep part only by pushing the inserting unit in, as the intestines are complicatedly bent and it is hard to convey force to the tip of the inserting unit. For example, when the endoscope is drawn to pick up the extra bending or slack of the endoscope which was made during the deep insertion, the tip of the inserting unit is also drawn and the bending and the slack is not picked up, which makes the deep insertion difficult.

An endoscope device adapted to prevent the tip of the inserting unit from being drawn when extra bending or slack of the endoscope is picked up by attaching a balloon to the tip peripheral part of the inserting unit of the endoscope and blowing up the balloon and temporally fixing it to the intestines are proposed.

In the conventional art, an endoscope device which can improve operational performance of the endoscope device by providing an overtube through which the inserting unit of the endoscope is inserted and attaching a balloon at the tip peripheral part of the overtube and blowing up or deflating the balloon of the overtube and the balloon of the endoscope as required is proposed. For example, the Japanese Patent Laid-Open No. 2002-301019 discloses an endoscope device adapted to supply air from a pump device, while control means measures an air pressure inside each of the balloon of the endoscope and the balloon of the overtube and controls a pressure inside each of the balloons.

In the endoscope device disclosed in the Patent Gazette, if the material of the balloon of the endoscope and the balloon of the overtube are different from each other or if material of a ballo on differs for each time the balloon is exchanged, the balloon blows up different ways even with the same amount of flow. Therefore, in order to keep a constant way of blowing up even if the materials and the type of the balloons are different, the amount of flow of gas sent to each of the balloons needs to be controlled according to the material of the balloon. In order to control the amount of flow of gas sent or a gas sending pressure to each of the balloons, a gas sending time to each of the balloons needs to be measured.

In a conventional art described in the abovementioned Japanese Patent Laid-Open No. 2002-301019, however, air pressures inside the balloon of the endoscope and the balloon of the overtube are controlled but an amount of flow of gas sent to each of the balloons cannot be controlled. In the abovementioned device, there is a problem in that a gas sending time or a gas drawing time to or from each of the balloons which is needed to control the amount of flow of gas sent or the gas sending pressure to each of the balloons cannot be measured.

The present invention is adapted in view of the circumstances, and it is an object of the invention to provide an endoscope balloon control device which can measure a gas sending time or a gas drawing time to or from a balloon of an endoscope and a balloon of an overtube.

### Disclosure of the Invention

### Means for Solving the Problem

In order to achieve the abovementioned object, the endoscope balloon control device of the present invention comprises: a pump for supplying or discharging gas to or from a balloon for fixing attached to a peripheral part of a tip part of an inserting unit of an endoscope; a time detecting unit for detecting a gas sending time or a gas drawing time to or from the balloon; and a control unit for controlling a pressure inside the balloon by measuring the gas pressure inside the balloon and operating the pump based on the measured result, and also controlling a pressure inside the balloon based on the detected result by the time detecting unit.

### Brief Description of the Drawings

Figure 1 is a configuration diagram showing an entire configuration of an endoscope system to which an endoscope balloon control device is applied according to a first embodiment of the present invention;
Figure 2 is a configuration diagram showing an outlined configuration of the endoscope balloon control device of Figure 1;
Figure 3 is a diagram showing a configuration example of the remote controller of Figure 2;
Figure 4 is a block diagram showing an inner configuration of the endoscope balloon control device of Figure 2;
Figure 5 to Figure 11 are for illustrating an operation state of an endoscope and an overtube by using respective balloons, and Figure 5 is an illustration showing a state where respective balloons are deflated and the endoscope is inserted through the overtube and inserted in the intestines;
Figure 6 is an illustration showing a state where a balloon for an overtube is blown up and fixed in the intestines;
Figure 7 is an illustration showing a state where the endoscope is further inserted into the overtube from the state of Figure 6;
Figure 8 is an illustration showing a state where the balloon of the endoscope is blown up and fixed to the wall of the intestines in the state of Figure 7;
Figure 9 is an illustration showing a state where the balloon of the overtube is deflated and the overtube is further inserted in the state of Figure 8;
Figure 10 is an illustration showing a state where the tip of the overtube moves to the tip part of the endoscope from the state of Figure 9;
Figure 11 is an illustration showing a state where the balloon of the overtube is blown up and fixed to the wall of the intestines in the state of Figure 10;
Figure 12 to Figure 15 are flowcharts for illustrating an operation of an endoscope balloon control device, and Figure 12 is a flowchart showing a main program of a control unit;
Figure 13 is a flowchart showing a processing routine based on the switch state checking module of Figure 12;
Figure 14 is a flowchart showing a processing routine based on the gas sending module of Figure 13;
Figure 15 is a flowchart for showing a processing routine based on the gas drawing module of Figure 13; and
Figure 16 is a block diagram showing an inner configuration of the endoscope balloon control device according to a second embodiment.

### Best Mode for Carrying Out the Invention

Embodiments of the present invention will be described with reference to the drawings.

### Embodiment 1

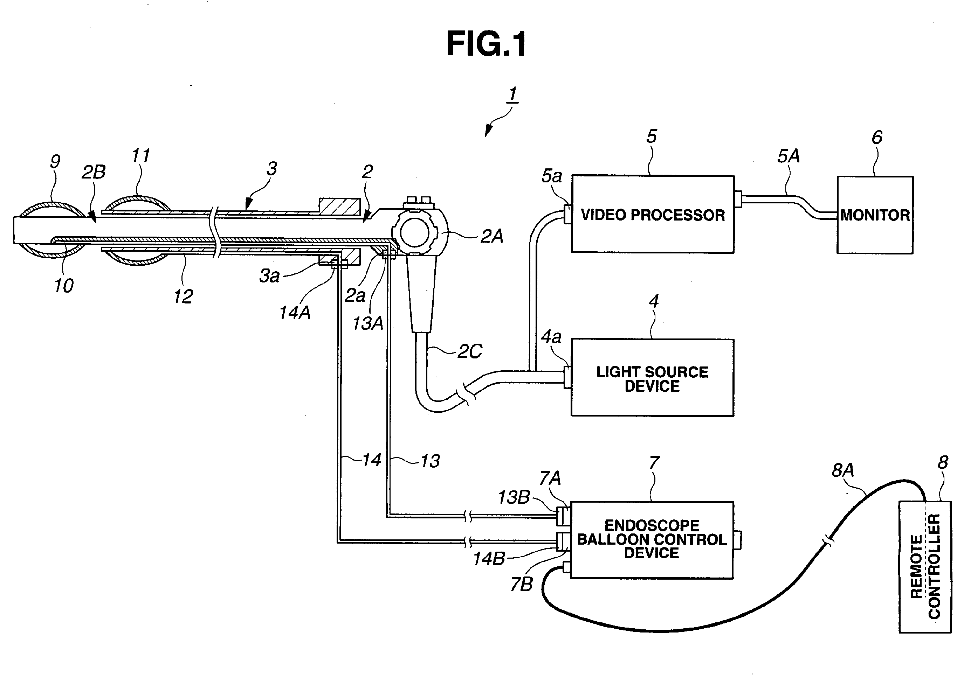

Figure 1 is a configuration diagram showing an entire configuration of an endoscope system applied with an endoscope balloon control device according to a first embodiment of the present invention.

As shown in Figure 1, an endoscope system 1 having an endoscope balloon control device of the embodiment has an endoscope 2, an overtube 3, a light source device 4, a video processor 5, a monitor 6, an endoscope balloon control device 7 and a remote controller 8.

The endoscope 2 is used for an alimentary canal endoscope examination, for example, having an inserting unit 2B to be inserted in a body cavity and an operation unit 2A provided at a base end side of the inserting unit 2B. An observation optical system including an illumination optical system and a CCD which is an image pickup device (not shown) is provided in the tip part of the inserting unit 2B and illuminates an observed part in the alimentary canal of a subject body and can obtain an observation image in the alimentary canal of the subject body.

A universal code 2C is extending out from the operation unit 2A. Inside the universal code 2C, a signal line and a light guide cable (not shown) are provided. The base end part of the universal code 2C is connected to a connector 4a of the light source device 4 and a connector 5a of the video processor 5. Accordingly, illumination light from the light source device 4 is supplied to the illumination optical system of the endoscope 2 via the light guide cable in the universal code 2C and illuminates the observed part, and outputs an image pick up signal in the alimentary canal outputted from the CCD to the video processor 5.

Such an endoscope 2 is adapted to be used inserted through the overtube 3 during an operation. A configuration of the overtube 3 will be described later.

The light source device 4 is a light source device for supplying illumination light to the illumination optical system provided for the endoscope 2 via a light guide (not shown) in the light guide cable.

The video processor 5 performs signal processing on an image pickup signal from the CCD of the endoscope 2 and supplies image data based on the image pickup signal (for example, endoscope live image data) to the monitor 6.

The monitor 6 is connected to the video processor 5 by a connecting cable 5A. The monitor 6 displays an endoscope image based on image data from the video processor 5.

In the endoscope system 1 of the embodiment, a fixing balloon 9 is attached to the tip peripheral part of the inserting unit 2B of the endoscope 2. An air supplying tube 10 provided along the inserting unit 2B from the base end part side to the tip part side of the inserting unit 2B is connected to the balloon 9.

The base end part of the operation unit 2A of the air supplying tube 10 is connected to the connector 2a provided at the bottom of the operation unit 2A. To the connector 2a is connected a connector 13A which is provided at one end of an endoscope balloon gas sending tube (hereinafter, referred to as the first gas sending tube) 13, the other end of which is connected to an endoscope balloon control device 7 to be described later. Thus, the balloon 9 is blown up by gas sent from the endoscope balloon control device 7 and temporally fixed to the alimentary canal such as the intestinal canal.

The overtube 3 is for guiding the inserting unit 2B to be inserted in the alimentary canal, for example, by inserting the endoscope 2 through it, and has a little bit bigger inside diameter than the external diameter of the inserting unit 2B of the endoscope. The overtube 3 has a configuration with the same flexibility as the inserting unit 2B of the endoscope 2. Further, to the tip peripheral part of the overtube 3, a tube fixing balloon 11 is attached.

To the balloon 11, an air supplying tube 12 provided for the overtube 3 from its base end part side to its tip part side is connected.

The base end part at the opposite side of the balloon 11 of the air supplying tube 12 (an inserting slot side for inserting the endoscope 2 of the overtube 3) is connected to a connector 3a provided near the inserting slot of the overtube 3. To the connector 3a is connected a connector 14A which is provided at one end of an overtube balloon gas sending tube (hereinafter referred to as a second gas sending tube) 14, the other end of which is connected to the endoscope balloon control device 7. Thus, the balloon 11 is blown up by gas sent from the endoscope balloon control device 7 and temporally fixed to the alimentary canal such as the intestine canal.

The endoscope balloon control device 7 is for controlling various operations on the amount of flow of gas sent of the balloon 9 of the endoscope 2 and the balloon 11 of the overtube 3 and the like.

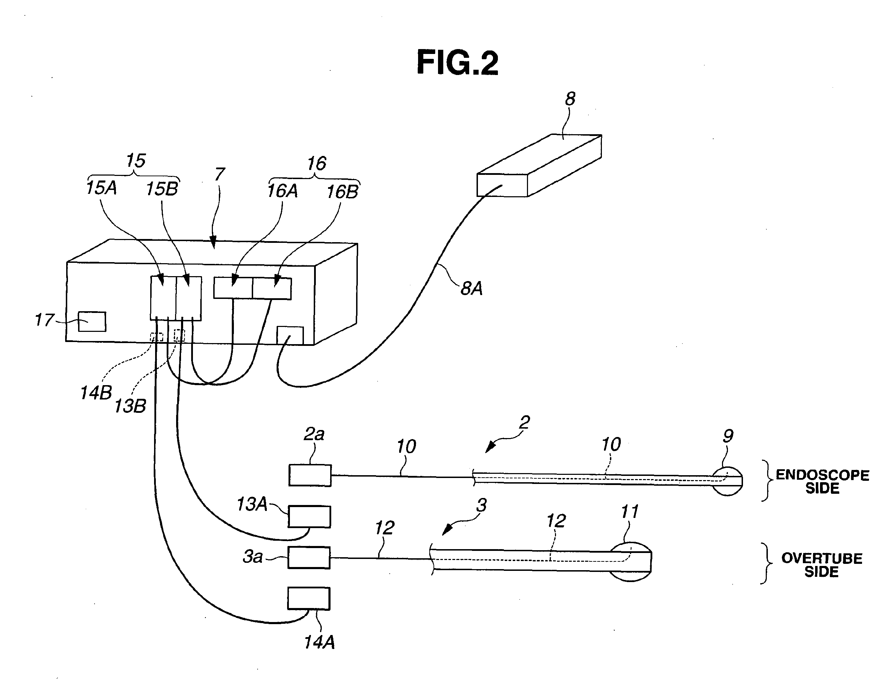

Figure 2 is a configuration diagram showing an outlined configuration of the endoscope balloon control device.

As shown in Figure 2, a backflow prevention tank 15 is provided for the endoscope balloon control device 7, and a pressure display device 16 and a power source switch 17 are provided at the front side of the device 7.

The backflow prevention tank 15 is adapted to be able to prevent backflow of a liquid, having a tank 15A for the balloon 9 of the endoscope 2 and a tank 15B for the balloon 11 of the overtube 3. The first and the second gas sending tubes 13 and 14 are connected to the corresponding tanks 15A and 15B respectively.

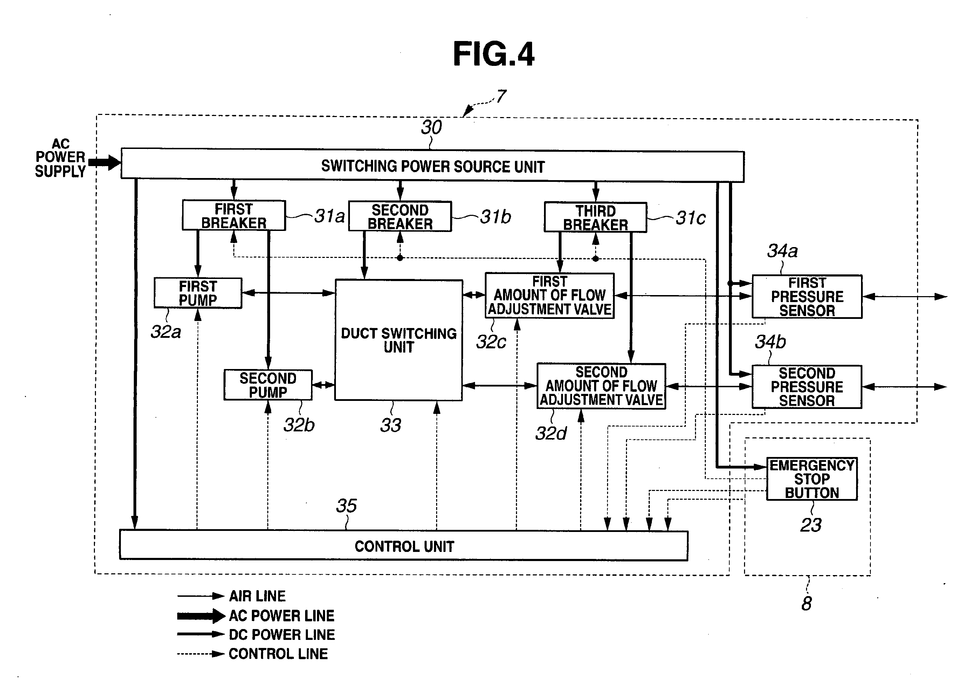

The tanks 15A and 15B send gas to the balloons 9 and 11 respectively via the first and the second gas sending tubes 13 and 14 by increasing the inside pressure via the first and the second pumps 32a and 32b (see Figure 4) to be described later by the control of the endoscope balloon control device 7, respectively. In such a case, the tanks 15A and 15B are adapted to prevent backflow of a liquid from the first and the second gas sending tubes 13 and 14 by a backflow prevention mechanism (not shown).

As such, a gas sending duct via the air supplying tube 10 connected to the balloon 9 of the endoscope 2, the first gas sending tube 13, and the tank 15A, a gas sending duct via the air supplying tube 12 connected to the balloon 11 of the overtube 3, the second gas sending tube 14, and the tank 15B are provided for the endoscope balloon control device 7 of the embodiment.

The pressure display device 16 is for displaying a pressure value of ducts connected to the balloons 9 and 11 by using a detecting device (not shown). The pressure display device 16 has a display device 16A for the balloon 9 of the endoscope 2 and a display device 16B for the balloon 11 of the overtube 3.

The display device 16A displays a pressure value inside the duct for the balloon 9 of the endoscope 2, and the display device 16B displays a pressure value inside the duct for the balloon 11 of the overtube 3.

The power source switch 17 is a switch for switching the power source of the endoscope balloon control device 7 between ON and OFF.

As shown in Figure 1 and Figure 2, the remote controller 8 is connected to a side of the endoscope balloon control device 7 via a connecting cable 8A. The remote controller 8 is electrically connected to a control unit 35 provide d inside the endoscope balloon control device 7 to be described later via the connecting cable 8A.

In the embodiment, the endoscope balloon control device 7 is adapted to be supplied with an operation signal for pressure control and gas sending control of each of the balloons 9 and 11 by an operator to manipulate the remote controller 8 during an operation.

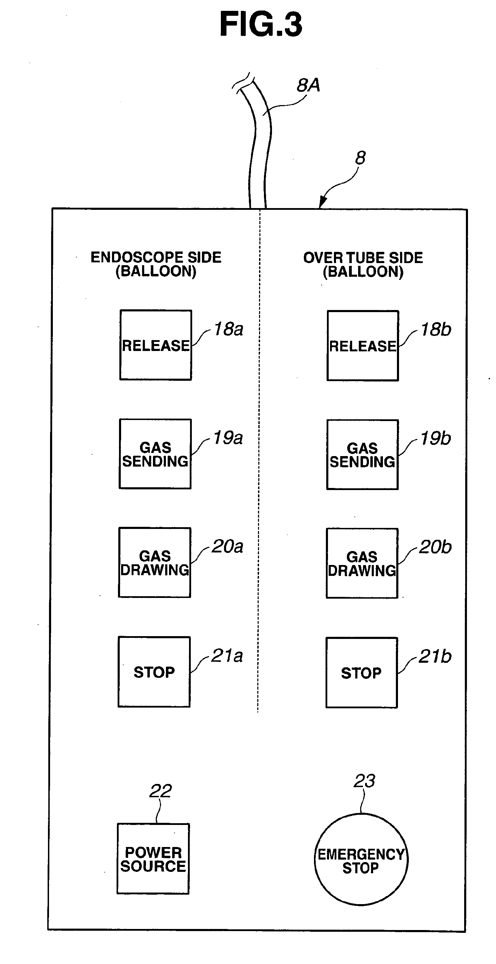

Figure 3 is a diagram showing a configuration example of the remote controller 8.

As shown in Figure 3, the remote controller 8 is set up as its various buttons for controlling an endoscope side balloon and various buttons for controlling an overtube side balloon are divided into right and left of the main body of the remote controller, for example, for facilitating manipulation of the operator.

At the left side of the remote controller 8, a release button 18a, a gas sending start button 19a, a gas drawing start button 20a and a stop button 21a are provided as operation buttons for controlling the endoscope side balloon.

To the right side of the remote controller 8, a release button 18b, a gas sending start button 19b, a gas drawing button 20b and a stop button 21b are provided as operation buttons for controlling the overtube side balloon.

Further, a power source button 22, and an emergency stop button 23 are provided at the bottom of the remote controller 8.

The release button 18a is a button for indicating to release air in a duct of the balloon 9 of the endoscope 2. The gas sending start button 19a is a button for indicting to start sending gas into the balloon 9 of the endoscope 2. The gas drawing start button 20a is a button for indicating to start drawing gas from the balloon 9 of the endoscope 2. The stop button 21a is a button for performing indication to stop a gas sending operation by the gas sending start button 19a or a gas drawing operation by the gas drawing start button 20a. As the stop button 21a is pressed down, an air pressure inside the balloon 9 can be kept.

On the other hand, the release button 18b is a button for indicating to release air inside the duct of the balloon 11 of the overtube 3. The gas sending start button 19b is a button for indicating to start sending gas into the balloon 11 of the overtube 3. The gas drawing start button 20b is a button for indicating to start drawing gas from the balloon 11 of the overtube 3. The stop button 21b is a button for performing indication to stop a gas sending operation by the gas sending start button 19b and a gas drawing operation by the gas drawing start button 20b. As the stop button 21b is pressed down, an air pressure inside the balloon 11 or a duct can be kept.

The power source switch 22 is a button for switching the power source of the endoscope balloon control device 7 between the ON state and the OFF state.

The emergency stop button 23 is a button for directly turning off the first to the third breaker 31a to 31c to be described later of the endoscope balloon control device 7 and perform emergency stop on gas sending control of each of the balloons 9 and 11 by the endoscope balloon control device 7.

Next, an inner configuration of the endoscope balloon control device 7 will be described with reference to Figure 4. Figure 4 is a block diagram showing an inner configuration of the endoscope balloon control device.

As shown in Figure 4, the endoscope balloon control device 7 has a switching power source unit 30, a first to a third breakers 31a to 31c, a first and a second pumps 32a, 32b, a first and a second amount of flow adjustment valves 32c, 32d, a duct switching unit 33, a first and a second pressure sensors 34a, 34b, and a control unit 35 which is the control means.

To the switching power source unit 30, AC power is supplied from an external commercial power source via a connecting code (not shown). The switching power source unit 30 converts the supplied AC power into DC power and supplies it to the first to the third breakers 31a to 31c, the first and the second pressure sensors 34a and 34b, the control unit 35 and the emergency stop button 23 of the remote controller 8.

The first breaker 31a is electrically connected to the first and the second pumps 32a and 32b and the emergency stop button 23 of the remote controller 8. The first breaker 31a supplies DC power to the first and the second pumps 32a and 32b. If an operation signal is supplied from the emergency stop button 23, supplying of DC power to the first and the second pumps 32a and 32b is stopped.

The second breaker 31b is electrically connected to the duct switching unit 33 and the emergency stop button 23 of the remote controller 8. The second breaker 31b supplies DC power to the duct switching unit 33. If an operation signal is supplied from the emergency stop button 23, supplying of DC power to the duct switching unit 33 is stopped.

The third breaker 31c is electrically connected to the first and the second amount of flow adjustment valves 32c and 32d and the emergency stop button 23 of the remote controller 8. The third breaker 31c supplies DC power to the first and the second amount of flow adjustment valves 32c and 32d. If an operation signal is supplied from the emergency stop button 23, supplying of DC power to the first and the second amount of flow adjustment valves 32c and 32d is stopped.

The first and the second pumps 32a and 32b are connected to an input side of the duct switching unit 33 via an air line, respectively. The first and the second pumps 32a and 32b are adapted to be controlled for driving based on a control signal from the control unit 35. For example, they are adapted to s end air to the duct switching unit 33 via the air line, or, on the contrary, to draw air from the duct switching unit 33 via the air line.

To the output side of the duct switching unit 33, the first and the second amount of flow adjustment valves 32c and 32d are connected via an air line, respectively. The first and the second amount of flow adjustment valves 32c and 32d are valves which can be adjusted between open and close by the control unit 35, and makes adjustment of the amount of flow of air to be output based on a control signal from the control unit 35. The first and the second amount of flow adjustment valves 32c and 32d supply air by the adjusted amount flow within a predetermined range to the first and the second pressure sensors 34a and 34b via a gas sending line, respectively.

The fist and second pressure sensors 34a and 34b measure air pressures supplied from the first and the second amount of flow adjustment valves 32c and 32d. In the embodiment, measured results by the first and the second pressure sensors 34a and 35b may be supplied to the control unit 35 and the control unit 35 may be adapted to control the first and the second pumps 32a and 32b to make them desired air pressures based on the supplied measured results respectively.

Outputs from the first and the second pressure sensors 34a and 34b are adapted to be supplied to the first and the second gas sending tubes 13 and 14 via a gas sending line, connectors 7A, 7B, 13B and 14B, respectively.

As such, the endoscope balloon control device 7 has a gas sending duct comprising the first amount of flow adjustment valve 32c and the first pressure sensor 34a via the fist pump 32a and the duct switching unit 33, and a gas sending duct comprising the second amount of flow adjustment valve 32d and the second pressure sensor 34b via the second pump 32b and the duct switching unit 33.

The duct switching unit 33 can switch ducts (not shown) provided inside so as to make it a duct state according to an executing mode in the endoscope balloon control device 7. For example, as the executing mode, there are four executing modes such as a gas sending mode, a gas drawing mode, a keeping mode, and a releasing mode. Therefore, the duct switching unit 33 can switch an inner duct (not shown) to make it a state according to the four modes, i.e., a gas sending state, a gas drawing state, a keeping state, and a releasing state. The switching can be controlled based on a control signal from the control unit 35. As a result, it is adapted to make the duct at the balloon 9 side of the endoscope 2 and the duct at the balloon 11 side of the overtube side 3 connected to the back side duct states based on respective desired executing modes.

Although they are not shown, the control unit 35 has an amount of flow counter for counting the amount of flow of gas sent to the balloon 9 of the endoscope 2 and the balloon 11 of the overtube 3; a timer counter, which is the gas sending time detecting means for counting each of a gas sending time and a gas drawing time of the balloons 9 and 11 or a timer for measuring a predetermined time; and a storing unit storing a main program to be described later or a program based on various modules.

The control unit 35 is adapted to control the first and the second pumps 32a and 32b, and the duct switching unit 33 and the first and the second amount of flow adjustment valves 32c, 32d by using the amount of flow counter and the timer counter by executing the program based on an operation signal from the remote controller 8.

Then, the endoscope balloon control device 7 is adapted to be able to measure a gas sending time or a gas drawing time to or from the balloon 9 of the endoscope 2 and the balloon 11 of the overtube 3, and time of the amount of flow of gas sent and the like, and control the amount of flow of gas sent to the balloon 9 of the endoscope 2 and the balloon 11 of the overtube 3 by using the measured result.

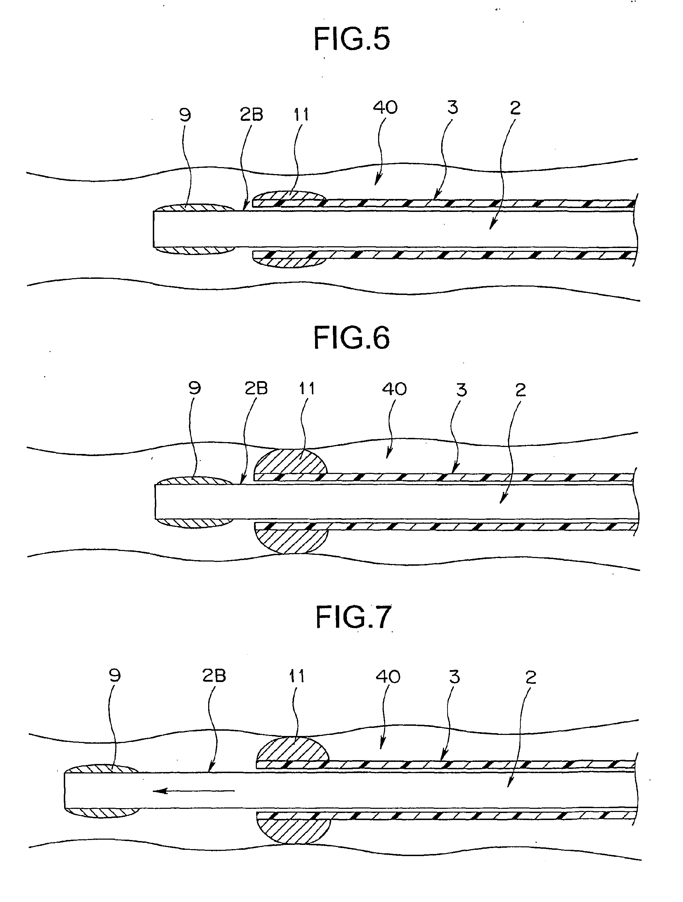

Next, a basic operation state of the endoscope system 1 will be described with reference to Figure 5 to Figure 11.

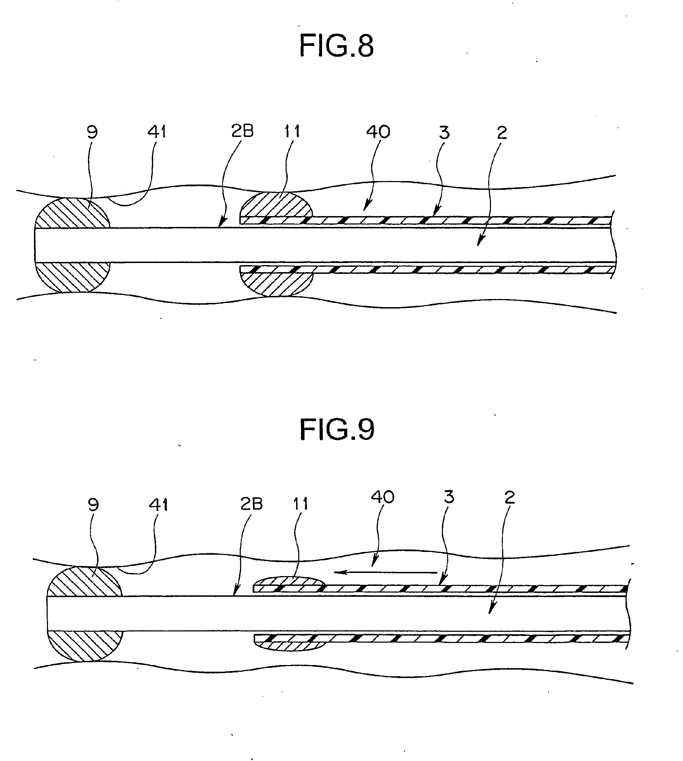

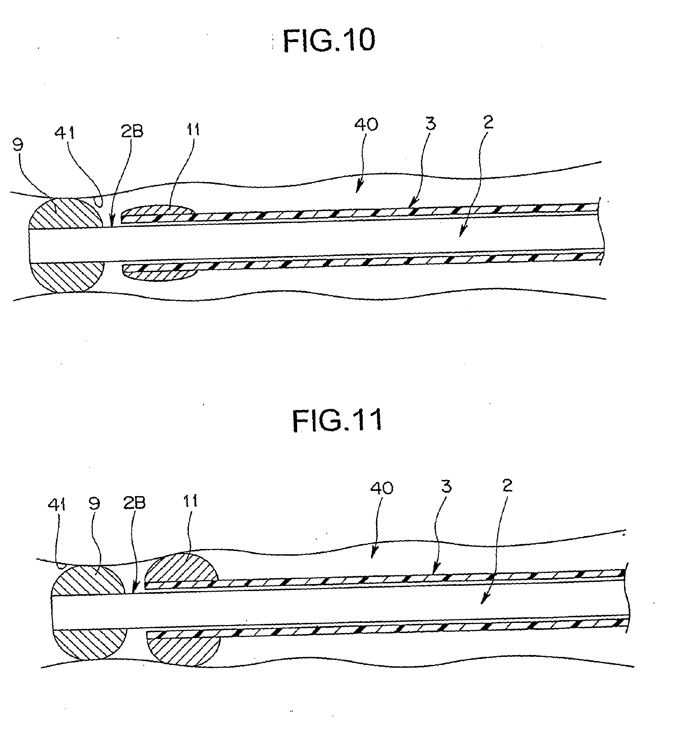

From Figure 5 to Figure 11 are illustrations describing a n operation state of an endoscope 2 and an overtube by using the balloon of the endoscope and the balloon of the overtube. Figure 5 shows a state where each balloon is deflated and an endoscope is inserted through the overtube and inserted into the intestines; Figure 6 shows a state where the balloon of the overtube is blown up and fixed to the intestines; Figure 7 is a state where the endoscope is further inserted into the overtube from the state of Figure 6; Figure 8 shows a state where the balloon of the endoscope is blown up and fixed to the intestine wall in the state of Figure 7; Figure 9 shows a state where the balloon of the overtube is deflated and the overtube is further inserted in the state of Figure 8; Figure 10 shows a state where the tip of the overtube moves to the endoscope tip from the state of Figure 9; and Figure 11 shows a state where the balloon of the overtube is blown up and fixed to the intestine wall in the state of Figure 10, respectively.

As shown in Figure 5, an operator inserts the endoscope 2 into the overtube 3. In such a case, the balloon 9 of the endoscope 2 and the balloon 11 of the overtube 3 are in a deflated state as air inside are drawn respectively, and the operator starts insertion of the endoscope 2 to the subject in this state.

Next, when the operator inserted the tip of the endoscope 2 and the overtube 3 to, for example, a descending leg of duodenum, the operator presses down the gas sending start button 19b (see Figure 3) at the overtube side of the remote controller 8 to supply air into the balloon 11 for fixing the main body attached to the tip of the overtube 3 from the second pump 32b, blow up the balloon 11 and fix it to the intestines 40 of the overtube 3 as shown in Figure 6. Next, the operator keeps the overtube 3 to the intestines 40 and inserts only the inserting unit 2B of the endoscope 2 into the deep part as shown in Figure 7.

Then, the operator presses down the gas sending start button 19a (see Figure 3) at the endoscope side of the remote controller 8 in a state where the inserting unit 2B of the endoscope 2 is inserted by a predetermined distance to supply air into the balloon 9 for fixing the main body attached to the tip of the endoscope 2 from the first pump 32a, blow up the balloon 9 and fix to the intestine wall 41 as shown in Figure 8.

Some patients may have narrow intestines 40, 41 however, the operator can optimally blow up each of the balloons 9 and 11 and fix it to the intestines 40, 41 according to the size of the intestines 40, 41 only by pressing down the stop buttons 21a and 21b to stop air supply to each of the balloons 9 and 11.

Next, the operator presses down the release button 18b or the gas drawing start button 20b (see Figure 3) at the overtube side of the remote controller 8 to release air in the balloon 11 by the duct switching unit 33 or draw air in the balloon 11 of the overtube 3 from the second pump 32b and deflate the balloon 11 (see Figure 9).

Next, the operator inserts the overtube 3 to the deep part along the endoscope 2 and inserts the tip of the overtube 3 near the tip of the inserting unit 2B of the endoscope 2 as shown in Figure 9.

Then, the operator presses down the gas sending start button 19b (see Figure 3) at the overtube side of the remote controller 8 in the state where the tip of the overtube 3 is inserted near the tip of the inserting section 2B to supply air to the balloon 11 of the overtube 3 from the second pump 32b, blow up the balloon 11 and fix the overtube 3 to the intestines wall, as shown in Figure 11.

The operator presses down the release button 18a or the gas drawing start button 20a at the endoscope side of the remote controller 8 (see Figure 3) to release air in the balloon 9 by the duct switching unit 33 or draw air in the balloon 9 of the endoscope 2 from the first pump 32a and deflate the balloon 9 and further insert the inserting unit 2B to the deep part.

As the manipulations from Figure 5 to Figure 11 are repeated, insertion of the endoscope 2 and the overtube 3 into the deep part is advanced, so that the inserting unit 2B of the endoscope 2 can be inserted to a desired place.

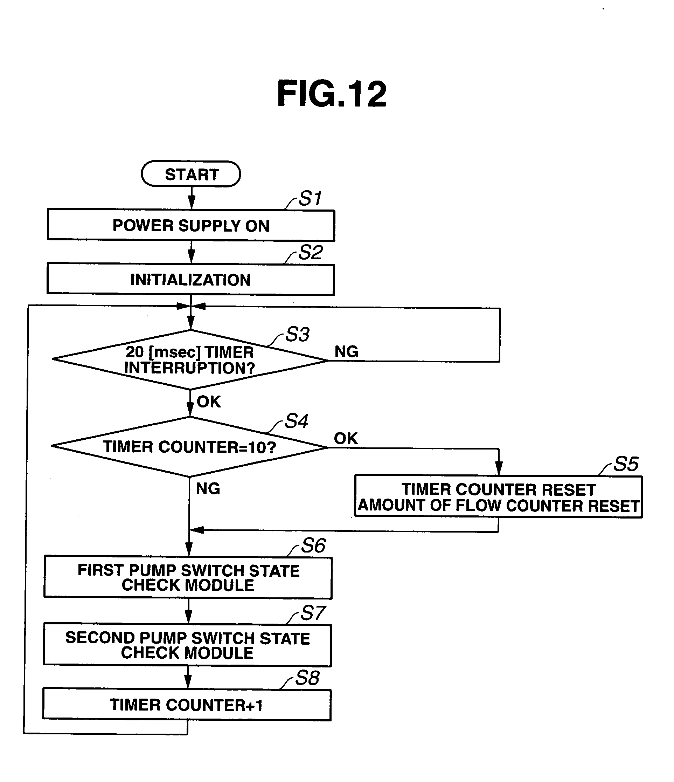

Next, operations of the endoscope balloon control device of the embodiment will be described with reference to Figure 12 to Figure 15.

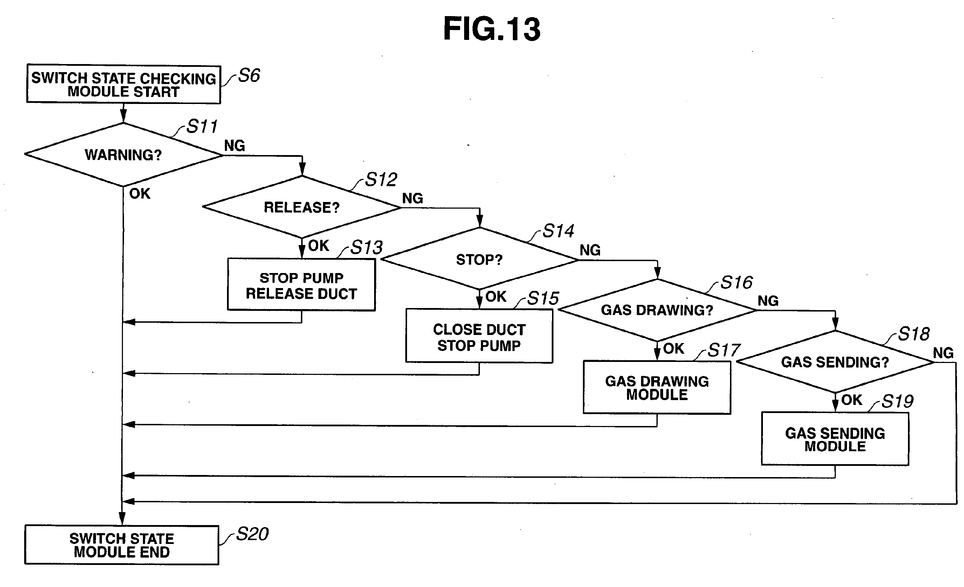

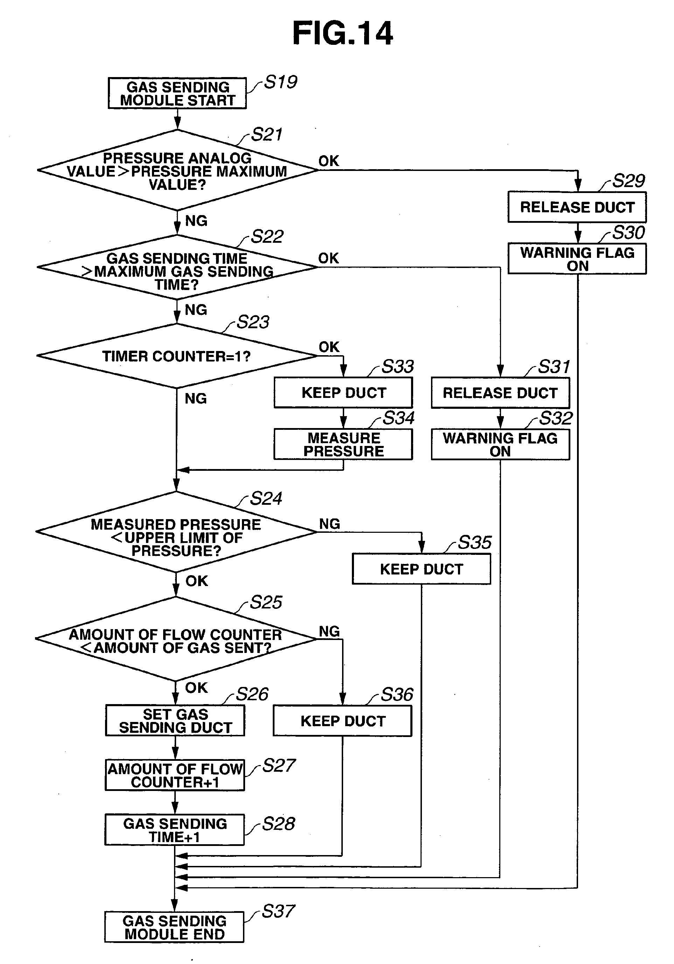

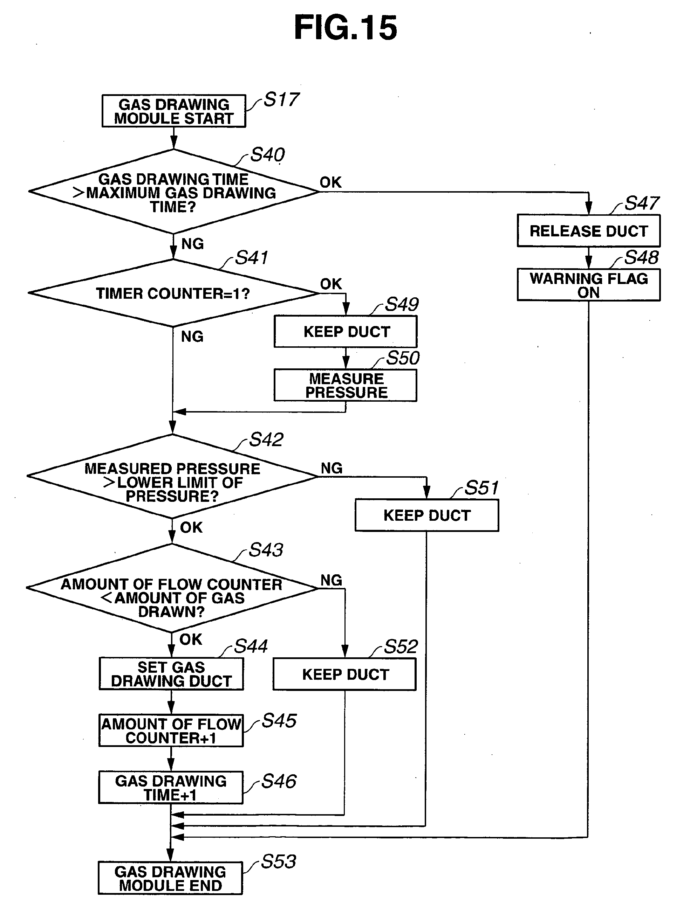

From Figure 12 to Figure 15 are for describing operations of the endoscope balloon control device and; Figure 12 is a flowchart for showing a main program of a control unit; Figure 13 is a flowchart for showing a processing routine based on a switch state checking module of Figure 12; Figure 14 is a flowchart for showing a processing routine based on a gas sending module of Figure 13; and Figure 15 is a flowchart for showing a processing routine based on a gas drawing module of Figure 13.

It is assumed that an operator performs an endoscope examination in the alimentary canal by using the endoscope system 1 of Figure 1. When the operator presses down a power source button 22 of the remote controller 8 shown in Figure 3 (or a power source switch 17 shown in Figure 2), the control unit 35 reads in a main program shown in Figure 12 from a storing unit (not shown) inside and starts it.

When the control unit 35 checks the ON state of the power source by the process at the step S1, it performs initialization on various appliances and the like in the endoscope balloon control device 7 by the process at the step S2. As the initialization, for example, the control unit 35 starts the first and the second pumps 32a and 32b and performs initialization so that it is in a duct release state by the duct switching unit 33. The control unit 35 initializes by resetting an amount of flow counter, a timer count and the like (not shown) in the control unit 35.

Then, the control unit 35 determines 20 msec timer interruption by the determination process at the following step S3; and if it determines that it occurred, it transfers the process to the step S4; and if it determines that it did not occur, it keeps performing the determination process.

For the timer, one for measuring 20 msec is used for operating the processing routine by every 20 msec shown in Figure 12.

Then, the control unit 35 determines whether the counter value of the timer counter for counting one by every 20 msec of the timer is equal to 10 or not by the determination process at the step S4; and if it determines that they are equal, it resets the timer counter and the amount of flow counter by the process at the step S5 and transfers the process to the step S6. On the other hand, if it determines that the counter value is not equal to 10, the control unit 35 transfers the process to the step S6.

In the embodiment, it is assumed that balloon control is performed according to manipulation of various buttons by the operator using the controller 8 by ten times of the processing routine shown in Figure 12, i.e., by the time unit of 200 msec.

Next, the control unit 35 executes the processing routine based on the first pump and the second pump switch state checking module to be described later by the processes at the step S6 and the step S7. Then, after the processing routine based on the switch state checking module has been performed, the control unit 35 adds 1 to the counter value by the timer counter at the process at the step S8 and then returns the process to the determination process at the step S3.

Next, the processing routine of the first pump switch state checking module by the step S6 will be described with reference to Figure 13.

When the control unit 35 executes the process at the step S6, it starts the processing routine of the switch state checking module shown in Figure 13.

Then, the control unit 35 determines the presence of warning by whether the emergency stop button 23 of the remote controller 8 has been pressed down or not by the determination process at the step S11. In such a case, the control unit 35 performs the determination process by detecting ON/OFF of the warning flag. The warning flag is adapted to be turned ON not only by the emergency stop button 23 being pressed down but also by the control unit 35 when the endoscope balloon control device 7 has a problem and enters into a warning state.

Here, when it is detected that the warning flag is turned ON, the control unit 35 determines that the endoscope balloon control unit 7 is in a warning state because of pressing down of the emergency stop button 23 or the like, and ends the processing routine based on the switch state checking module by the process at the step S20 to keep the current warning process and transfers to the process at the step 7 of Figure 12.

On the other hand, if it is detected that the warning flag is OFF by the determination process at the step S11, the control unit 35 determines that the emergency stop button 23 is not pressed down and the endoscope balloon control device 7 is not in a warning state, and transfers to the determination process at the step S12.

At the determination process at the step S12, the control unit 35 determines whether the release button 18a of the remote controller 8 shown in Figure 3 is pressed down or not, and if it determines that it is not pressed down, it transfers to the step S14. On the other hand, if it determines that it is pressed down, the control unit 35 stops an operation of the first pump 32a corresponding to the release button 18a by the process at the step S13 and controls the duct switching unit 33 and the first amount of flow adjustment valve 32c so as to release the duct, and then it transfers to the step S20 and ends the processing routine based on the first pump switch state checking module.

In the determination process at the step S14, the control unit 35 determines whether the stop button 21a of the remote controller 8 shown in Figure 3 is pressed down or not, and if it determines that it is not pressed down, it transfers to the step S16. On the other hand, if it determines that it is pressed down, the control unit 35 controls the duct switching unit 33 and the first amount of flow adjustment valve 32c so as to close the duct (a duct keeping state) by the process at the step S15, and also stops the operation of the first pump 32a corresponding to the stop button 21a, and then transfers to the step S20 and ends the processing routine based on the first pump switch state checking module.

In the determination process at the step S16, the control unit 35 determines whether the gas drawing starting button 20a of the remote controller 8 shown in Figure 3 is pressed down or not; and if it determines that it is not pressed down, it transfers to the step S18. On the other hand, if it determines that it is pressed down, the control unit 35 starts the processing routine based on the gas drawing module shown in Figure 15 to be described later in the process at the step S17, performs gas drawing control corresponding to the gas drawing start button 20a, and then transfers to the step S20 and ends the processing routine based on the first pump switch state checking module.

In the determination process at the step S18, the control unit 35 determines whether the gas sending start button 19a of the remote controller 8 shown in Figure 3 is pressed down or not; and if it determines that it is not pressed down, it transfers to the step S20. On the other hand, if it determines that it is pressed down, the control unit 35 starts the processing routine based on a gas sending module shown in Figure 14 to be described later in the process at the step S19, and performs the gas sending control corresponding to the gas sending start button 19a, and then transfers to the step S20 and ends the processing routine based on the first pump switch state checking module.

Next, the processing routine of the gas sending module at the step S19 will be described with reference to Figure 14.

In the embodiment, the control unit 35 is adapted to previously set the pressure maximum value and the pressure upper limit value for comparing them with a pressure measured results from the fist and the second sensors 34a and 34b. The pressure upper limit value and the pressure maximum value fulfill relationship of the pressure limit value < the pressure maximum value. In such a case, the pressure maximum value means the pressure value equal to cause each of the balloons 9 and 11 to be blown up to a dangerous state, and the pressure upper limit value means a pressure value equal to cause each of the balloons 9 and 11 to be blown up and fixed to the intestines.

When the control unit 35 executes the process at the step S19, it starts the processing routine of the gas sending module shown in Figure 14.

Then, the control unit 35 compares a measured result from the first pressure sensor 34a (for example, a pressure analog value) and the previously set pressure maximum value in the determination process at the step S21; and if it determines that the measured result is smaller than the pressure maximum value, it transfers to the step S22; and if it determines that it is bigger, it transfers to the process at the step S29. In the process at the step S29, the control unit 35 stops the operation of the first pump 32a and also controls the duct switching unit 33 and the first amount of flow adjustment valve 32c so as to release the duct. Then, the control unit 35 turns on the warning flag in the process at the step S30, then it transfers to the step S37 and ends the processing routine based on the gas sending module.

In the process at the step S22, the control unit 35 compares a measured result of measuring a gas sending time by an inner timer (gas sending time) and a previously set maximum gas sending time; and if it determines that the gas sending time is shorter than the maximum gas sending time, it transfers to the step 23; and if it determines that the gas sending time is longer than the maximum gas sending time, it transfers to the process at the step S31.

In the process at the step S31, the control unit 35 stops the operation of the first pump 32a as in the process at the step S29, and controls the duct switching unit 33 and the first amount of flow adjustment valve 32c so as to release the duct. Then, the control unit 35 turns on the warning flag in the process at the step S32 as in the process at the step S30, then transfers to the step S37 and ends the processing routine based on the gas sending module.

Then, the control unit 35 determines whether the counter value of the timer counter for counting one for every 20 msec of the timer is equal to 1 or not in the determination process at the step S23; and if it determines that it is equal, it stops the operation of the first pump 32a in the process at the step S33 and also controls the duct switching unit 33 and the first amount of flow adjustment valve 32c to keep the duct state. Then, the control unit 35 obtains a measured pressure value by performing pressure measurement by the first and the second pressure sensors 34a and 34b in the process at the step S34 and transfers to the step S24.

In the determination process at the step S23, if it determines that the counter value of the timer counter is not equal to 1, the control unit 35 transfers to the step S24.

Then, the control unit 35 obtains a measured pressure value by performing pressure measurement by the first pressure sensor 34a and compares the obtained measured pressure value (the measured pressure value obtained at the step S34 if it did via a loop of the steps S33 and S34) and a previously set pressure upper limit value in the determination process at the step S24; and if it determines that the measured result is smaller than the pressure upper limit value, it transfers to the step S25; and if it determines that the measured result is bigger than the pressure upper limit value, it transfers to the process at the step S35.

In the process at the step S35, the control unit 35 stops the operation of the first pump 32a and also controls the duct switching unit 33 and the first amount of flow adjustment valve 32c so as to keep the duct state, then it transfers to the step S37 and ends the processing routine based on the gas sending module.

Next, the control unit 35 compares the amount of flow counter value and a predetermined amount of flow of gas sent previously set (amount of flow of gas sent) in the determination process at the step S25; and if it determines that the amount of flow counter value is bigger than the predetermined amount of gas sent, it stops the operation of the first pump 32a in the process at the step S36 and also controls the duct switching unit 33 and the first amount of flow adjustment valve 32c so as to keep the duct state, and then it transfers to the step S37 and ends the processing routine based on the gas sending module.

The amount of flow counter value is a variable for determining how much amount of gas can be sent in 200 msec (20 msec x 10), and can be set from 0 to 9 according to the timer counter value from 0 to 9, for example. Actually, the amount of flow counter (not shown) counts the amount of flow counter value for every 20 msec. The predetermined amount of gas sent indicates an approximate amount of gas sent (amount of gas sent) which can be sent for every 20 msec of the endoscope balloon control device 7.

If the amount of flow counter value is smaller than the predetermined amount of gas sent in the determination process at the step S25, the control unit 35 controls the duct switching unit 33 to set a gas sending duct based on the gas sending start buttons 19a and 19b in the process at the step S27. Then, the control unit 35 adds 1 to the amount of flow counter value in the process at the step S27 and then adds 1 to the gas sending time in the process at the following step S28, and transfers to the step S37 and ends the processing routine based on the gas sending module.

When the processing routine based on the gas sending module ends, the control unit 35 executes the process based on the second pump switch state checking module at the step S7 shown in Figure 12 assuming that the processing routine based on the switch state checking module shown in Figure 13 finished, and thereafter repeatedly executes the processes according to the processing routine shown in Figure 12. Only the operation of the second pump switch state checking module, and a pump, a switch and an object of control are changed, thus, the description of them will be omitted.

Next, the processing routine of the gas drawing module at the step S17 shown in Figure 13 will be described with reference to Figure 15.

When the control unit 35 executes the process of the step S17, it starts the process routine of a gas drawing module shown in Figure 15.

Then, the control unit 35 compares the measured result of the gas drawing time measured by the inner timer (a gas drawing time) and a previously set maximum gas drawing time in the determination process at the step S40; and if it determines that the gas drawing time is shorter than the maximum gas drawing time, it transfers to the step S41; and if it determines that the gas drawing time is longer than the maximum gas drawing time, it transfers to the process at the step S47.

In the process at the step S47, the control unit 35 stops the operation of the first pump 32a and also controls the duct switching unit 33 and the first amount of flow adjustment valve 32c to release the duct. Then, the control unit 35 turns on the warning flag in the process at the step S48 and then transfers to the step S53 and end s the processing routine based on the gas drawing module.

Then, the control unit 35 determines whether the counter value of the timer counter for counting 1 for each 20 msec of the timer is equal to 1 or not in the determination process at the step S41; and if it determines that it is equal, it stops the operation of the first pump 32a in the process at the step S49 and also controls the duct switching unit 33 and the first amount of flow adjustment valve 32c to keep the duct state. Then, the control unit 35 obtains a measured pressure value by performing pressure measurement by the first pressure sensor 34a in the process at the step S50, and transfers to the step S42.

In the determination process at the step S41, if it determines that the counter value of the timer counter is not equal to 1, the control unit 35 transfers to the step S42.

Then, the control unit 35 obtains a measured pressure value by performing pressure measurement by the first pressure sensor 34a and compares the obtained measured pressure value (the measured pressure value obtained at the step S50 if it did via a loop of the steps S49 and S50) and a previously set pressure lower limit value in the determination process at the step S42; and if it determines that the measured result is smaller than the pressure lower limit value, it transfers to the step S51; and if it determines that the measured result is bigger than the pressure lower limit value, it transfers to the process at the step S43.

In the process at the step S51, the control unit 35 stops the gas drawing operation of the first and the second pumps 32a and 32b and also controls the duct switching unit 33 and the first and the second amount of flow adjustment valves 32c and 32d so as to keep the duct, then it transfers to the step S53 and ends the processing routine based on the gas drawing module.

Next, the control unit 35 compares the amount of flow counter value and a predetermined amount of gas drawn previously set (amount of gas drawn) in the determination process at the step S43; and if it determines that the amount of flow counter value is bigger than the predetermined amount of gas drawn, it stops the gas drawing operation of the first pump 32a in the process at the step S52 and also controls the duct switching unit 33 and the first amount of flow adjustment valve 32c so as to keep the duct state, and then it transfers to the step S54 and ends the processing routine based on the gas drawing module. The predetermined amount of gas drawn indicates an approximate amount of gas drawn (amount of flow of air drawn) which can be drawn for every 20 msec of the endoscope balloon control unit 7.

If it determines that the amount of flow counter value is smaller than the predetermined amount of gas drawn in the determination process at the step S43, the control unit 35 controls the duct switching unit 33 to set a gas drawing duct based on the gas drawing start button 20a in the process at the step S44. Then the control unit 35 adds 1 to the amount of flow counter value in the process at the step S45 and adds 1 to the gas drawing time in the process at the following step S46, and transfers to the step S53, and ends the processing routine based on the gas drawing module.

When the processing routine based on the gas drawing module ends, the control unit 35 executes the process based on the first pump control module at the step S7 shown in Figure 12 assuming that the processing routine based on the switch state checking module shown in Figure 13 finished, and thereafter repeatedly executes the process according to the processing routine shown in Figure 12.

As mentioned above, according to the embodiment, as the endoscope balloon control device 7 can measure a gas sending time to the balloon 9 of the endoscope 2 and the balloon 11 of the overtube 3, it can control a pressure inside a balloon so as to release a duct by detecting that the maximum gas sending time, the maximum gas drawing time, the maximum gas sending pressure and the maximum gas drawing pressure are exceeded so that a procedure can be performed without applying great deal of force to the intestine wall.

As the endoscope balloon control device 7 can adjust the amount of flow of gas sent and an amount of flow of gas drawn to or from each of the balloons 9 and 11, it can be adapted to various materials of balloons or various parts. The endoscope balloon control device 7 can prevent a serial gas sending operation or a serial gas drawing operation which occurs when a duct such as the first and the second gas sending tubes 13 and 14 and the like, for example, come off.

In the embodiment, although a configuration of the remote controller 8 connected to the endoscope balloon control device 7 has been described, the present invention is not limited to that and can be configured with the operation unit 2A of the endoscope 2, which is at hand of the operator, or a foot switch for controlling the endoscope balloon control device 7 at the foot of the operator, for example.

The controller 8 may be adapted to send various remote control operation signals using infrared radiation, receive the infrared radiation by a photoreceptor provided for the endoscope balloon control device 7 and capture the remote control signal. That further facilitates an operator's manipulation.

### Embodiment 2

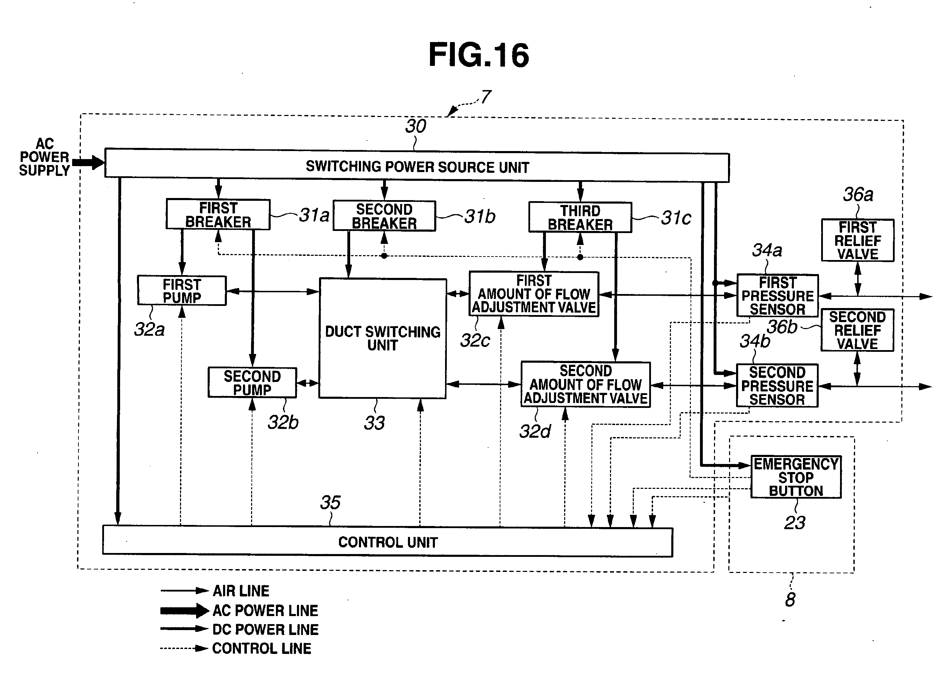

Figure 16 is a configuration diagram showing an outlined configuration of the endoscope balloon control device according to the second embodiment of the present invention. Figure 16 gives the same reference numerals to the same components as those of the endoscope balloon control device of the first embodiment and omits the description of them, thus, only different parts will be described.

The endoscope balloon control device 7 of the embodiment provides the first and the second relief valves 36a and 36b as pressure inside the balloon control means on gas sending ducts (gas sending lines) of two systems.

As shown in Figure 16, the first relief valve 36a is connected to a duct at an output terminal side of the first pressure sensor 34a. The second relief valve 36b is connected to a duct at an output terminal side of the second pressure sensor 34b.

The first and the second relief valves 36a and 36b are for preventing a pressure of a duct from being more than a predetermined pressure by monitoring pressure of ducts, and when the pressure of the duct becomes more than a predetermined pressure (a relief pressure), releasing the duct to the outside.

In the embodiment, for the first and the second relief valves 36a and 36b, mechanical relief valves are used, for example, and the mechanical relief valves are adapted to release a valve fixed with elastic materials to the outside as the pressure inside the duct increases.

The other configurations are the same as those in the first embodiment.

The operation of the embodiment will be described with reference to Figure 16.

In the endoscope balloon control device 7 shown in Figure 16, the respective relief pressures are set so as to satisfy,
pressure upper limit value < pressure maximum value =< relief pressure.

Generally, the pressure upper limit value and the pressure maximum value are controlled by the electric type first and the second pressure sensors 34a and 34b which are pressure control methods different from the pressure inside the balloon control means (the first and the second relief valves 36a and 36b). An electromagnetic valve for adjusting the amount of flow (not shown) included in the first and the second amount of flow adjustment valves 32c and 32d is also an electric type and controlled by the control unit 35.

Therefore, as the first and the second pressure sensors 34a and 34b and the first and the second amount of flow adjustment valves 32c and 32d are both an electric type, if an electric system in the device breaks down, for example, both of the fist and the second pressure sensors 34a and 34b and the first and the second amount of flow adjustment valves 32c and 32d may stop their operations.

In such a case, outputs from the first and the second pumps 32a and 32b are directly applied into a living body so that a great deal of pressure may be applied. Even if an operation of a pressure sensor and an amount of flow adjustment valve corresponding to one of the gas sending ducts stops, an output from a corresponding pump may be directly applied into a living body.

The endoscope balloon control device 7 of the embodiment, however, decreases the applied pressure into a living body as the mechanical first and the second relief valves 36a and 36b which can operate regardless of the electric system failure, if a pressure to the living body exceeds a relief pressure in the abovementioned failure.

Even if an operation of a pressure sensor and an amount of flow adjustment valve corresponding to a gas sending duct of one of the gas sending ducts of two systems (gas sending lines) stops but a pressure to a living body exceeds a relief pressure, the endoscope balloon control device 7 can decrease an applied pressure into a living body and also can normally operate the other gas sending duct as a corresponding relief valve operates.

The other operations are the same as those of the first embodiment.

Therefore, according to the embodiment, the same effects as those of the first embodiment are obtained and an endoscope balloon control device having pressure inside the balloon control means which can ensure safety also to failure such as in an electric system can be realized.

In the present invention, it is apparent that different embodiments in a wide range can be adapted based on the present invention without departing from the spirit and the scope of the present invention. The present invention is not limited by particular embodiments except for being limited by the range of the attached claims.

### Industrial Applicability

The endoscope balloon control device of the present invention is effective particularly in observation, treatment or the like on cases or various parts performed by changing materials of a balloon, as it can measure a gas sending time or a gas drawing time to a balloon of an endoscope and a balloon of an overtube so that an amount of flow of gas sent or a gas sending pressure to each of the balloons can be controlled.

## Claims

1. An endoscope balloon control device **characterized by** comprising:
a pump for supplying or discharging gas to or from a ballo on for fixing attached to a peripheral part of a tip part of an inserting unit of an endoscope;
a time detecting unit for detecting a gas sending time or a gas drawing time to or from the balloon; and
a control unit for controlling a pressure inside the balloon by measuring the gas pressure inside the balloon and operating the pump based on the measured result, and also controlling a pressure inside the balloon based on the detected result by the time detecting unit.

2. The endoscope balloon control device according to claim 1, **characterized by** comprising:
a pump for supplying or discharging gas to or from a balloon for fixing attached to a peripheral part of a tip part of an overtube for the endoscope being inserted therethrough; wherein the time detecting unit detects a gas sending time or a gas drawing time to or from the balloon of the endoscope and the balloon of the overtube.

3. The endoscope balloon control device according to claim 1, **characterized by** comprising an amount of flow detecting unit for detecting the amount of flow of gas sent or drawn to or from the balloon, and controlling the amount of flow of gas sent or drawn to or from the balloon by operating the pump based on the detected result by the amount of flow detecting unit.

4. The endoscope balloon control device according to claim 2, **characterized by** comprising an amount of flow detecting unit for detecting the amount of flow of gas sent or drawn to or from a balloon of the endoscope and a balloon of the overtube, and controlling the amount of flow of gas sent or drawn to or from the balloon by operating the pump based on the detected result by the amount of flow detecting unit.

5. The endoscope balloon control device according to claim 1, **characterized in that** the control unit stops an operation of the pump when the detected result from the time detecting means exceeds a set threshold.

6. The endoscope balloon control device according to claim 2, **characterized in that** the control device stops an operation of the pump when the detected result from the time detecting means exceeds a set threshold.

7. The endoscope balloon control device according to claim 1, **characterized in that** the control unit controls a pressure inside the balloon between a first pressure value and a second pressure value, and when the pressure inside the balloon exceeds the second pressure value, which is bigger than the first pressure value, decreases the pressure inside the balloon.

8. The endoscope balloon control device according to claim 2, **characterized in that** the control unit controls pressures inside the balloon of the endoscope and the balloon of the overtube between the first pressure value and the second pressure value, and when the pressure inside the balloon exceeds the second pressure value, which is bigger than the first pressure value, decreases the pressure inside the balloon.

9. The endoscope balloon control device according to claim 3, **characterized in that**, even when a failed control method occurs among a plurality of control methods for controlling a pressure inside the balloon, the control unit can control by the remaining control method.

10. The endoscope balloon control device according to claim 4, **characterized in that**, even when a failed control method occurs among a plurality of control method s for controlling pressures inside the balloon of the endoscope and the balloon of the overtube, the control unit can control by the remaining control method.

11. The endoscope balloon control device according to claim 1, **characterized in that** the control unit has a relief valve for releasing to outside when a pressure inside a duct from the pump to the balloon exceeds a set value.

12. The endoscope balloon control device according to claim 2, **characterized in that** the control unit has a relief valve for releasing to outside when a pressure inside a duct from each of the pumps to each of the balloons exceeds a set value.

13. An endoscope balloon control device **characterized by** comprising:
a pump for supplying or discharging gas to or from a balloon for fix ing attached to a peripheral part of a tip part of an inserting unit of an endoscope; and
a control unit for controlling a pressure inside the balloon by measuring the gas pressure inside the balloon and operating the pump based on the measured result;
wherein the control unit comprises an amount of flow detecting unit for detecting the amount of flow of gas sent or drawn to or from the balloon, and a time detecting unit for detecting a gas sending time or a gas drawing time to or from the balloon, and operates the pump and controls the amount of gas sent or drawn to or from the balloon based on the detected result by the amount of flow detecting means unit and the detected result by the time detecting unit.

14. An endoscope balloon control device **characterized by** comprising:
a pump for supplying or discharging gas to or from a balloon for fixing attached to a peripheral part of a tip part of an inserting unit of an endoscope and a balloon for fixing attached to a peripheral part of a tip part of an overtube for the endoscope being inserted therethrough and performs guiding in inserting of the endoscope; and
a control unit for controlling a pressure inside each of the balloons by measuring the gas pressure inside each of the balloons and operating the pump based on the measured result;
wherein the control unit comprises an amount of flow detecting unit for detecting the amount of flow of gas sent or drawn to or from the balloon, and a time detecting unit for detecting a gas sending time or a gas drawing time to or from the balloon, and operates the pump and controls the amount of gas sent or drawn to or from each of the balloons based on the detected result by the amount of flow detecting unit and the detected result by the time detecting unit.

15. The endoscope balloon control device according to claim 13, **characterized in that** the control unit stops an operation of the pump when the detected result from the amount of flow detecting unit and the detected result from the time detecting unit exceed a set threshold.

16. The endoscope balloon control device according to claim 14, **characterized in that** the control unit stops an operation of the pump when the detected result from the amount of flow detecting unit and the detected result from the time detecting unit exceed a set threshold.

17. The endoscope balloon control device according to claim 13, **characterized in that** the time detecting unit is a timer counter provided in the control unit.

18. The endoscope balloon control device according to claim 14, **characterized in that** the time detecting unit is a timer counter provided in the control unit.

19. The endoscope balloon control device according to claim 13, **characterized by** comprising a remote controller for controlling the control unit.

20. The endoscope balloon control device according to claim 14, **characterized by** comprising a remote controller for controlling the control unit.

21. The endoscope balloon control device according to claim 19, **characterized in that** the remote controller comprises a plurality of operation buttons and an emergency stop button for giving instructions to the control unit; wherein the control unit stops an operation of the pump when the emergency stop button is manipulated.

22. The endoscope balloon control device according to claim 20, **characterized in that** the remote controller comprises a plurality of operation buttons and an emergency stop button for giving instructions to the control unit; wherein the control unit stops an operation of the pump when the emergency stop button is manipulated.

23. The endoscope balloon control device according to claim 13, **characterized in that** the control unit has a relief valve for releasing to outside when a pressure inside a duct from the pump to the balloon exceeds a set value.

24. The endoscope balloon control device according to claim 14, **characterized in that** the control unit has a relief valve for releasing to outside when a pressure inside a duct from each of the pumps to each of the balloons exceeds a set value.
